# EUROPÄISCHE PATENTSCHRIFT

(11) **EP 3 615 725 B1**
(45) Veröffentlichungstag und Bekanntmachung des Hinweises auf die Patenterteilung: **09.06.2021**
(21) Anmeldenummer: 18719802.3
(22) Anmeldetag: 17.04.2018
(51) Int. Cl.: D06F 31/00, D06F 35/00, D06F 39/00

(54) **UV BEHANDLUNG IN DER INDUSTRIELLEN WÄSCHEREI AUF TAKTWASCHANLAGEN (WASCHSTRASSEN)**
UV TREATMENT IN INDUSTRIAL LAUNDRY ON INTERMITTENT WASHING SYSTEMS (TUNNEL WASHERS)
TRAITEMENT PAR RAYONNEMENT UV EN BLANCHISSERIE INDUSTRIELLE SUR DES TUNNELS DE LAVAGE (LIGNES DE LAVAGE)

(30) Priorität: 25.04.2017 DE 102017206924
(43) Veröffentlichungstag der Anmeldung: 04.03.2020
(73) Patentinhaber: CHT Germany GmbH, 72072 Tübingen (DE)
(72) Erfinder: STEVENS, Prince Charles, 89174 Altheim (DE)
(74) Vertreter: dompatent von Kreisler Selting Werner - Partnerschaft von Patent- und Rechtsanwälten mbB
(86) Internationale Anmeldenummer: PCT/EP2018/059707
(87) Internationale Veröffentlichungsnummer: WO 2018/197253

(56) Entgegenhaltungen:
- WO-A1-03/016608
- WO-A1-2014/146165
- JP-A- 2006 230 957

## Beschreibung

Die vorliegende Erfindung betrifft ein Verfahren zur Reinigung von Textilien in einer Taktwaschanlage unter Einsatz von UV-Strahlung, eine Vorrichtung zur Durchführung des Verfahrens sowie die Verwendung von UV-Strahlung, insbesondere UVC-Strahlung, in Waschverfahren von Textilien in Taktwaschanlagen.

In der industriellen Wäscherei werden im Wesentlichen zwei verschiedene Arten von Waschmaschinen eingesetzt: Wasch-Schleuder-Maschinen und Taktwaschanlagen. Die letztgenannten werden auch als Waschstraßen oder seltener als Postenwaschanlagen beziehungsweise CBW=continous batch washer bezeichnet. Wasch-Schleuder-Maschinen zeichnen sich dadurch aus, dass sie nur eine Waschkammer in Form einer Waschtrommel enthalten. Die Wäsche wird, wie bei einer haushaltsüblichen Waschmaschine, in die Trommel eingebracht, dort mit einer Waschflotte gewaschen und anschließend durch Schleudern entwässert. Damit arbeiten Wasch-Schleuder-Maschinen prinzipiell diskontinuierlich.

Demgegenüber ermöglichen Taktwaschanlagen eine kontinuierliche Fahrweise. Den Kern einer Taktwaschanlage bildet beispielsweise ein langes Metallrohr, das in mehrere Kammern aufgeteilt ist. Die zu waschenden Textilien werden in Form einzelner Textilposten auf einer Seite des Rohrs in die Taktwaschanlage eingebracht. Die Textilposten werden in einem festgelegten Takt von Kammer zu Kammer bewegt. In jedem Takt kann auf der einen Seite des Rohrs ein weiterer Textilposten eingebracht werden. Gleichzeitig kann in jedem Takt hinter dem Rohr ein gereinigter Textilposten entnommen werden. Die Anzahl der in der Taktwaschanlage befindlichen Textilposten entspricht damit der Anzahl der Kammern, die Verweilzeit eines Textilpostens in der Taktwaschanlage, der Anzahl der Kammern, multipliziert mit der Dauer eines Taktes. Die Aufteilung der Taktwaschanlage in Kammern kann erfolgen, indem die Anlage in ihrem Inneren beispielsweise eine Archimedische Schraube enthält. Die Schraube kann auch den Transport der Textilposten von Kammer zu Kammer bewirken. Weitere Möglichkeiten zum Postentransport sind beispielsweise der sogenannte. Dry-transfer, oder bottom transfer, beziehungsweise Überwurfsystem.

Taktwaschanlagen lassen sich im Prinzip in vier Sektionen aufteilen:
1. Sektion: Vorwäsche
2. Sektion: Haupt- oder Klarwäsche
3. Sektion: Spülzone
4. Sektion: Neutralisierungs- und Ausrüstungskammer

Auf der Seite (Auslauf) der Taktwaschanlage, auf der die gewaschenen Textilposten entnommen werden, wird Frischwasser in die Anlage eingebracht und im Gegenstrom der Spülzone zu den Textilposten durch die Anlage transportiert. Die Textilposten kommen dadurch zu Beginn der Reinigung mit dem am stärksten verunreinigten Wasser in Berührung, während das frischeste Wasser zum Ende des Reinigungsvorgangs zum Einsatz kommt. In die einzelnen Kammern der Taktwaschanlage können Waschflotten, die Wasch- und Hilfsmittel enthalten, eingebracht werden. Zumindest in die letzte Kammer sollte keine Waschflotte eingebracht werden, damit der Textilposten durch das Frischwasser von den Wasch- und Hilfsmitteln gereinigt werden kann. Hinter der letzten Kammer schließt sich in der Regel eine Entwässerungseinheit, beispielsweise in Form einer Presse oder Schleuder an, in der die Textilposten entwässert werden. Das dabei anfallende Wasser kann aufbereitet und als sogenanntes. Rückgewinnungswasser im System wiederverwendet werden.

Gegenüber Wasch-Schleuder-Maschinen zeichnen sich Taktwaschanlagen durch eine kontinuierliche Arbeitsweise aus, mit größerer Kapazität, kürzere Waschzeit und verbesserter Effizienz. Dem steht ein höherer Investitionsaufwand entgegen. Eine Reihe von Möglichkeiten sind vorgeschlagen worden, um die Waschleistung von industriellen Waschmaschinen zu verbessern.

So beschreibt beispielsweise WO 2014/031478 A1 ein Verfahren zum Reinigen von Textilien, insbesondere in Taktwaschanlagen. Die Waschflotte enthält ein Waschmittel sowie ein halogenhaltiges Bleichmittel. Die derart behandelten Textilien werden anschließend mit einer wässrigen Lösung, die Persäuren enthält, in Kontakt gebracht. Auf diese Weise wird die Reinigungsleistung verbessert.

Aus WO 2010/144744 A2 ist eine Taktwaschanlage bekannt, die ein System zur Herstellung von Ozon umfasst. Das Ozon kann in eine oder mehrere der Kammern der Taktwaschanlage eingebracht werden. Ozon hat dabei eine desinfizierende Wirkung.

Zur Verbesserung der Reinigungsleistung von Wasch-Schleuder-Maschinen, ist auch die Verwendung von UV-Strahlung im Stand der Technik bekannt. So offenbart DE 10 2014 213 312 A1 eine Waschmaschine mit Entfärbungseinrichtung und Reservoir für wasserunlösliche Teilchen. Aus einer Waschkammer wird eine Waschflotte in eine Entfärbungseinrichtung geleitet, dort mit UV-Strahlung bestrahlt und anschließend zurück in die Waschkammer geführt. Die Waschflotte kann Wasserstoffperoxid enthalten, das durch die UV-Strahlung zu OH-Radikalen umgesetzt wird.

Die Kombination aus UV-Strahlung und Wasserstoffperoxid ist auch aus EP 1 363 987 B1 bekannt. Dort wird ein Verfahren zur Reinigung von Curcuma-Flecken beschrieben. Textilien, wie Teppiche oder andere Stoffe, werden einer wässrigen Wasserstoffperoxidlösung ausgesetzt und mit UV-Licht bestrahlt. Um die Textilien nicht der schädigenden Wirkung kurzwelliger UV-Strahlung auszusetzen, wird bevorzugt langwellige UV-Strahlung im UVA-Bereich eingesetzt.

Das Dokument WO 03/016608 A1 offenbart ein Verfahren zum Waschen von Textilien in einer Taktwaschanlage umfassend zwei oder mehr Kammern, wobei die Textilien in einem festgelegten Takt von Kammer zu Kammer transportiert werden, im Gegenstrom Wasser durch die Taktwaschanlage transportiert wird und in eine oder mehrere Kammern eine halogenfreie Waschflotte zugeführt wird, wobei die Waschflotte teilweise oder vollständig mit UV-Strahlung bestrahlt wird. Zudem offenbart WO 03/016608 A1 die Verwendung von UV-Strahlung in einem Reinigungsverfahren für Textilien in einer Taktwaschanlage.

Die aus dem Stand der Technik bekannten Taktwaschanlagen weisen eine Reihe von Nachteilen auf. So führt die Verwendung aggressiver Chemikalien, wie halogenhaltiger Bleichmittel oder Ozon zu einer Belastung der Umwelt. Ebenfalls umweltbelastend sind die typischerweise erforderlichen hohen Temperaturen. Höhere Temperaturen gehen zudem mit einem höheren Energieverbrauch einher.

Am wirtschaftlichsten sind derzeit Waschverfahren auf Basis von Wasserstoffperoxid. Diese konnten bislang jedoch nur erfolgreich bei Waschtemperaturen oberhalb von 70 °C eingesetzt werden, da insbesondere Wasserstoffperoxid in den im Stand der Technik bekannten Verfahren erst ab dieser Temperatur eine optimale Bleich- und Desinfektionswirkung sowie wirksame Fleckentfernung aufweist. Zudem ist unter Umständen der Zusatz von Natriumhypochlorit notwendig. Aus diesem Grund werden im Stand der Technik für Waschtemperaturen unterhalb von 70 °C wesentlich kritischere Chemikalien, wie beispielsweise Peressigsäure, Peroktansäure oder Pernonansäure, eingesetzt. Auch ε-Phthalamid-Peroxo-Capronsäure (PAP) findet bei 50 °C Einsatz, wobei hier sowohl die Kosten als auch das Waschergebnis nicht wirtschaftlich sind.

Der vorliegenden Erfindung liegt somit die Aufgabe zugrunde, Möglichkeiten bereitzustellen, die Waschleistung von Taktwaschanlagen zu verbessern bzw. die Verwendung umweltschädlicher Chemikalien sowie hoher Temperaturen einzuschränken. Insbesondere soll eine Möglichkeit bereitgestellt werden, Waschverfahren auf Basis von halogenfreien Waschflotten bei Temperaturen unter 70 °C effektiv einsetzen zu können.

Die erfindungsgemäße Aufgabenstellung wird in einer ersten Ausführungsform gelöst durch ein Verfahren zum Waschen von Textilien mit einer Waschflotte in einer Taktwaschanlage, das dadurch gekennzeichnet ist, dass die Waschflotte Wasserstoffperoxid und/oder Persäure in einem Lösungsmittel, insbesondere Wasser, umfasst.

Erfindungsgemäß umfasst die Taktwaschanlage zwei oder mehr Kammern. Die zu waschenden Textilien werden in einem festgelegten Takt von Kammer zu Kammer transportiert. Im Gegenstrom zu den Textilien wird Wasser durch die Taktwaschanlage geführt. Die halogenfreie Waschflotte, die mit UV-Strahlung bestrahlt wird, wird in eine oder mehrere Kammern der Taktwaschanlage zugeführt. Das Verfahren kann auch weitere Schritte umfassen, die zum Waschen von Textilien in Taktwaschanlagen im Stand der Technik bekannt sind.

"Halogenfrei" im Sinne der vorliegenden Erfindung schließt die Anwesenheit von halogenhaltigen Wasch- und Bleichmitteln, beispielsweise Natriumhypochlorit, aus.

Unter UV-Strahlung wird hier, in Übereinstimmung mit der Definition der Weltgesundheitsorganisation (WHO), elektromagnetische Strahlung mit einer Wellenlänge im Bereich von 1 nm bis 400 nm, insbesondere 100 bis 400 nm, verstanden. Das Spektrum der UV-Strahlen wird weiter untergliedert in UVA- (315 nm bis 380 nm), UVB- (280 nm bis 315 nm) und UVC-Strahlung (100 nm bis 280 nm). Das UV-Spektrum unterhalb von 100 nm wird als extremes UV bezeichnet. Unterhalb einer Wellenlänge von 0,25 nm beginnt das Spektrum der Röntgenstrahlung.

Durch die erfindungsgemäße Bestrahlung der Waschflotte mit UV-C-Strahlung werden Bestandteile der Waschflotte zersetzt, sodass freie Radikale entstehen. Diese freien Radikale führen zu einer verbesserten Reinigungsleistung. Der Einsatz an schädlichen Chemikalien sowie die Waschtemperatur können folglich ohne Verlust an Waschleistung reduziert werden. Durch die gebildeten Radikale werden die Bleichwirkung sowie die Fähigkeit zur Fleckentfernung der Waschflotte verbessert. Auch die Verweilzeit in den einzelnen Kammern der Taktwaschanlage lässt sich reduzieren. In Abhängigkeit von der Masse der zu reinigenden Textilposten können beispielsweise Taktzeiten von 80 bis 240 Sekunden realisiert werden. Ein Textilposten kann dabei beispielsweise eine Masse von 25 bis 100 kg, insbesondere 35 bis 75 kg, aufweisen. Es ist zudem möglich, die verwendeten Bestandteile der Waschflotte optimal auszunutzen.

Durch die erfindungsgemäße Kombination aus physikalischen und chemischen Effekten gestaltet sich ein sehr effizienter und nachhaltiger Waschprozess mit völlig neuen Dimensionen auch im Hinblick auf die sogenannten Nachwaschquoten. Da sich selbst hartnäckige Verfleckungen nun im ersten Waschdurchgang entfernen lassen, entfällt die kostenintensive Nachwäsche zum größten Teil. Die gebildeten freien Radikale können auch desinfizierend wirken. Diese sind im Handel erhältlich. Auf diese Weise kann eine gefürchtete Verkeimung der Waschstraße vermieden werden, was zu einer sehr umfangreichen hygienischen Sanierung der gesamten Maschine führen würde.

Das erfindungsgemäße Verfahren weist zudem den Vorteil auf, dass es sich sehr leicht auf Grundlage von kommerziell erhältlichen Taktwaschanlagen ausführen lässt. Hierzu ist lediglich die Integration eines UV-Reaktors erforderlich, mit entsprechender Flottenführung. Auf diese Weise kann ohne besonderen Aufwand und insbesondere ohne hohe Investitionskosten, die Effizienz bereits bestehender Taktwaschanlagen erheblich gesteigert werden. Abgesehen vom Verfahrensschritt des Einsatzes von UV-Strahlung, lassen sich im Wesentlichen etablierte Waschverfahren einsetzen.

Überraschenderweise hat sich gezeigt, dass sich durch die erfindungsgemäße Bestrahlung der Waschflotte mit UV-Strahlung in Kombination mit einer Taktwaschanlage besondere Vorteile ergeben. So macht sich das erfindungsgemäße Verfahren zunutze, dass die Textilien ohnehin nur kurze Zeit in einer Kammer der Taktwaschanlage verbringen. Insbesondere ist diese Verweilzeit deutlich kürzer als bei Wasch-Schleuder-Maschinen. Die mittels UV-C-Strahlung gebildeten Radikale haben im Allgemeinen nur eine kurze Lebensdauer. Während in Wasch-Schleuder-Maschinen der Effekt einer UV-Bestrahlung nur kurzzeitig und damit nur für einen Teil der Waschdauer wirkt, ist in Taktwaschanlagen die Verweildauer ohnehin so gering, dass die Strahlung nahezu für die volle Waschdauer zu einer verbesserten Reinigungsleistung führt. Taktwaschanlagen in Kombination mit UV-Strahlung bieten zudem den Vorteil, dass Waschflotten in verschiedene Kammern eingebracht und diese Waschflotten auch separat bestrahlt werden können.

Die erfindungsgemäße Bestrahlung der Waschflotte kann unmittelbar in einer oder mehrerer Kammern der Taktwaschanlage erfolgen. Auf diese Weise kann eine Kammer vollständig oder teilweise bestrahlt werden. Die Bildung freier Radikale findet dann unmittelbar in der Kammer der Taktwaschanlage statt. Diese Vorgehensweise bietet den besonderen Vorteil, dass die desinfizierende Wirkung der UV-Strahlung selber unmittelbar auf die Textilien wirkt.

Die erfindungsgemäße Bestrahlung der Waschflotte kann aber auch außerhalb der Kammern der Taktwaschanlage mit einem in einem Nebenstrom befindlichen separaten UV-Reaktor erfolgen. Waschflotte kann dazu aus einem Tank zunächst durch einen UV-Reaktor geführt werden und anschließend bestrahlte Waschflotte in eine oder mehrere Kammern der Taktwaschanlage geleitet werden. Dabei kann Waschflotte auch nur teilweise bestrahlt werden. Waschflotte kann mit weiterer unbestrahlter Waschflotte in einem beliebigen Verhältnis vermischt und diese Mischung einer oder mehrerer Kammern der Taktwaschanlage zugeführt werden.

Es ist weiterhin möglich, bestrahlte Waschflotte in einen Tank zu führen und dort zeitweilig zu lagern. Bei dem Tank kann es sich auch um einen Tank handeln, der weitere unbestrahlte Waschflotte enthält. Waschflotte kann auch aus einer der Kammern der Taktwaschanlage, nachdem sie dort mit Textilposten in Kontakt gebracht wurde, durch einen UV-Reaktor geführt und dort bestrahlt werden. Im Folgenden kann Waschflotte erneut in eine der Kammern der Taktwaschanlage geführt werden oder auch in einem Tank gelagert werden.

Die Prozessführung ist jedoch nicht auf die genannten Varianten beschränkt.

Im Folgenden werden bevorzugte Ausführungsformen des erfindungsgemäßen Verfahrens beschrieben. Die Beschreibung ist beispielhaft und soll nicht den Umfang der Erfindung beschränken. Auch andere als die beschriebenen Ausführungsformen sind möglich und von der Erfindung umfasst.

Erfindungsgemäß umfasst die Waschflotte Wasserstoffperoxid oder Persäuren in einem Lösungsmittel. Bevorzugt ist das Lösungsmittel Wasser. Persäuren im Sinne der vorliegenden Erfindung umfassen insbesondere Peressigsäure, Peroktansäure, Pernonansäure und ε-Phthalamid-Peroxo-Capronsäure. Auch deren Gemische untereinander sowie mit Wasserstoffperoxid können erfindungsgemäß eingesetzt werden. Durch die erfindungsgemäße Bestrahlung beispielsweise der peroxidhaltigen Waschflotte mit UV-C-Strahlung entstehen OH-Radikale. Die gebildeten OH-Radikale verbessern die Waschleistung der Waschflotte. Zudem ermöglicht die Bestrahlung, die Ausnutzung des Wasserstoffperoxids zu verbessern, indem nicht verbrauchtes H₂O₂ zu OH-Radikalen umgesetzt wird. Dadurch wird die Bleich- und Desinfektionswirkung des Peroxids verbessert.

Durch die erfindungsgemäße Kombination aus einer Waschflotte auf Basis von Wasserstoffperoxid und der Bestrahlung mit UV-C-Strahlung wird es ermöglicht, eine Wasserstoffperoxid-basierte Waschflotte unterhalb von 70 °C einzusetzen. Zudem kann dabei auf einen Zusatz von Natriumhypochlorit verzichtet werden. Auch der Einsatz kritischer Chemikalien, wie beispielsweise Persäuren, kann umgangen werden. Auch bei einer Reduktion der Temperatur auf etwa 45 °C ist, im Vergleich zu Standardverfahren bei 70 °C ohne UV-Bestrahlung, mit dem erfindungsgemäßen Verfahren die Fleckentfernung verbessert, der Weißgrad erhöht und die Faserschädigung verringert. Wahlweise kann auch bei gegenüber den Standardverfahren gleichem Waschergebnis, der Einsatz an Wasserstoffperoxid um 10 bis 30 % reduziert werden. Beim Einsatz der oben genannten Persäuren lässt sich die Waschtemperatur drastisch vermindern.

Bevorzugt enthält die Waschflotte weitere Bestandteile, wie beispielsweise Tenside, Alkali, Komplexbildner, optische Aufheller und Bleichmittel. Die Waschflotte kann sämtliche für Waschflotten übliche Inhaltsstoffe enthalten, ohne darauf beschränkt zu sein. Die Inhaltsstoffe erzielen dabei ihre aus dem Stand der Technik bekannte Wirkung, die überraschenderweise jedoch durch die erfindungsgemäße UV-Bestrahlung nochmals gesteigert wird.

Als Tenside können insbesondere nichtionogene Tenside und/oder anionaktive Tenside auf Basis von Fettalkoholethoxylaten und/oder Polyglukosiden eingesetzt werden, ohne darauf beschränkt zu sein. Als Alkali kommen beispielsweise solche auf Basis von Natronlauge und/oder Kalilauge infrage, ohne darauf beschränkt zu sein. Als Komplexbildner können beispielsweise solche auf Basis von Polycarbonsäuren und/oder Phosphonaten sowie Zuckeracrylsäurepolymere und/oder Kammpolymere genannt werden, ohne darauf beschränkt zu sein. Die Waschflotte kann beispielsweise optische Aufheller auf Basis von Stilbenderivaten enthalten, ohne darauf beschränkt zu sein. Mögliche halogenfreie Bleichmittel sind solche auf Basis von Wasserstoffperoxid und/oder ohne darauf beschränkt zu sein.

Durch die oxidative UV-Bestrahlung wird insbesondere die Waschkraft nichtionogener Tenside deutlich gesteigert. Durch die Bestrahlung der kompletten Waschflotte mit all ihren Bestandteilen, ergibt sich also eine besondere Steigerung der Waschkraft. Gerade diese Kombination bewirkt ein einzigartiges Waschergebnis, insbesondere Fleckentfernung, unter UV-Einfluss.

In einer bevorzugten Ausführungsform wird die Waschflotte vor der Zuführung zu einer der Kammern der Taktwaschanlage bestrahlt. Die Bestrahlung erfolgt somit außerhalb der Kammern der Taktwaschanlage. Die Textilien kommen auf diese Weise nicht direkt mit der UV-Strahlung in Berührung. Diese Vorgehensweise zeichnet sich folglich durch den besonderen Vorteil aus, dass die Textilien nicht durch UV-Strahlung geschädigt werden können. Zudem ist die Ausführung des Verfahrens auf Grundlage kommerziell erhältlicher Taktwaschanlagen oder bereits bestehender Anlagen besonders einfach, da lediglich außerhalb der Anlage ein UV-Reaktor in die Vorrichtung zur Zuführung der Waschflotte integriert werden muss.

Die vor der Zuführung zu einer Kammer zu bestrahlende Waschflotte kann dabei beispielsweise aus einem Tank stammen. Die zu bestrahlende Waschflotte kann jedoch auch aus einer der Kammern stammen. Auf diese Weise wird eine bereits einer der Kammern zugeführte Waschflotte entnommen, außerhalb der Kammer bestrahlt und anschließend wieder einer Kammer zugeführt.

Bevorzugt umfasst die eingesetzte UV-Strahlung Strahlungsanteile im UVC-Bereich. Besonders bevorzugt umfasst die eingesetzte UV-Strahlung Strahlungsanteile in einem Bereich von [250] nm bis [270] nm, insbesondere 254 nm. Alternativ kann die eingesetzte UV-Strahlung auch ausschließlich aus UVC-Strahlung bestehen, bevorzugt aus Strahlung in einem Bereich von [254] nm, insbesondere [250] bis [270] nm.

Überraschenderweise hat sich gezeigt, dass Strahlungsanteile im UVC-Bereich eine besonders starke Aktivierung der Waschflotte bewirken. Diese Wirkung kann noch gesteigert werden, wenn die eingesetzte Strahlung ausschließlich aus UVC-Strahlung besteht. So lässt sich beispielsweise die Wirkung von Wasserstoffperoxid durch UVC-Strahlung um das 4.000-fache steigern. UVC-Strahlung weist zudem den besonderen Vorteil auf, dass sie etwa im Bereich einer Wellenlänge von 250 nm bis 270 nm, insbesondere im Bereich um 258 nm, einen stark keimtötenden bzw. bakteriziden Effekt aufweist. Diese Eigenschaft lässt sich darauf zurückführen, dass die kurzwellige und energiereiche UVC-Strahlung durch eine Schädigung der DNS die Aufrechterhaltung des Stoffwechsels und der Zellteilung stoppt, sodass derart geschädigte Zellen letztlich absterben. Dabei ist eine mutationsbedingte Resistenzbildung ausgeschlossen. Diese desinfizierende Wirkung nimmt mit steigender Komplexität der angegriffenen Organismen ab. So lassen sich Viren und Bakterien wesentlich leichter zerstören als beispielsweise Pilze oder Pilzsporen. Da UVC-Strahlen grundsätzlich keine festen Stoffe durchdringen, ist eine sichere Abschirmung jedoch einfach möglich.

Bevorzugt wird das Waschen der Textilien bei einer Temperatur von 40 °C bis 70 °C, insbesondere 50 °C, durchgeführt. Die gegenüber den aus dem Stand der Technik bekannten Verfahren reduzierte Temperatur verringert den Energieverbrauch und ermöglicht ein umweltschonendes Verfahren.

Waschflotte enthält bevorzugt Wasserstoffperoxid und/oder Persäuren in einer Konzentration von 1 bis 5, besonders bevorzugt von 1 bis 3, insbesondere von 1 bis 2 g/kg Wäsche.

In einer weiteren Ausführungsform wird die erfindungsgemäße Aufgabe gelöst durch eine Taktwaschanlage zur Durchführung des oben beschriebenen Verfahrens. Die erfindungsgemäße Taktwaschanlage umfasst zwei oder mehr Kammern, eine Vorrichtung zum getakteten Transport von Textilposten von Kammer zu Kammer, eine Vorrichtung zur Zuführung von Wasser sowie eine Vorrichtung zur Zuführung einer Waschflotte in eine oder mehrere Kammern umfassend einen oder mehrere Tanks sowie ein Transportsystem und ist dadurch gekennzeichnet, dass die Taktwaschanlage einen oder mehrere UV-Reaktoren umfasst.

Die Zuführvorrichtung für das Wasser ist so ausgestaltet, dass das Wasser im Gegenstrom zu den Textilposten durch die Kammern transportiert werden kann. Die UV-Reaktoren sind entweder so in das Transportsystem für die Waschflotte eingebettet, dass die Waschflotte ganz oder teilweise aus einem Tank oder einer der Kammern der Taktwaschanlage durch einen oder mehrere UV-Reaktor(en) in die Kammern der Taktwaschanlage geführt werden kann oder so angeordnet, dass der Innenraum einer oder mehrerer der Kammern der Taktwaschanlage teilweise oder vollständig mit UV-Strahlung bestrahlt werden kann.

Das Transportsystem dient dem Transport der Waschflotte aus einem Tank in die Kammern der Taktwaschanlage. In der Alternative, in der ein oder mehrere UV-Reaktoren in das Transportsystem für die Waschflotte eingebettet sind, kann die Waschflotte außerhalb der Kammern der Taktwaschanlage bestrahlt werden und anschließend die bestrahlte Waschflotte den Kammern zugeführt werden. Das Transportsystem kann aus Rohrleitungen, Schläuchen oder dergleichen bestehen. Diese können durch Ventile oder dergleichen unterbrochen sein, sodass sich der Fluss durch das Transportsystem steuern lässt.

Das Transportsystem kann auch Pumpen umfassen, die den Transport von Flüssigkeiten ermöglichen.

Die erfindungsgemäße Taktwaschanlage ist nicht auf die beschriebenen Bestandteile beschränkt, sondern kann auch weitere im Stand der Technik bekannte Bestandteile umfassen, die üblicherweise in Taktwaschanlagen eingebaut werden. Im Folgenden werden bevorzugte Ausführungsformen der erfindungsgemäßen Taktwaschanlage beispielhaft beschrieben. Die Beschreibung stellt keine Beschränkung der Erfindung dar. Auch weitere Ausführungsformen sind möglich und von der Erfindung umfasst.

In einer bevorzugten Ausführungsform der erfindungsgemäßen Taktwaschanlage ist einer oder mehrere der UV-Reaktoren geeignet, UV-Strahlung, insbesondere im UVC-Bereich zu erzeugen. Die Reaktoren können zur Erzeugung von UV-Strahlung in einem breiteren Wellenlängenbereich, der den UVC-Bereich zumindest teilweise umfasst, geeignet sein. Die Reaktoren können auch geeignet sein, ausschließlich Strahlung im UVC-Bereich zu erzeugen.

Das Transportsystem ist vorzugsweise so beschaffen, dass die vor Zuführung zu einer der Kammern der Taktwaschanlage in einem UV-Reaktor bestrahlte Waschflotte wieder zurück in den Tank geführt werden kann. Bestrahlte Waschflotte kann auf diese Weise zeitweilig zwischengelagert und zu einem späteren Zeitpunkt eingesetzt werden. Diese bevorzugte Ausführungsform der Vorrichtung ermöglicht auch, bestrahlte Waschflotte in einem Tank mit unbestrahlter Waschflotte zu mischen. Dies kann dadurch ermöglicht werden, dass ein Rohr, Schlauch oder dergleichen von einem UV-Reaktor zurück zu einem Tank führt. Vorzugsweise ist die Verbindung durch ein Ventil unterbrochen.

In einer weiteren bevorzugten Ausführungsform ist das Transportsystem so beschaffen, dass die vor Zuführung zu einer der Kammern der Taktwaschanlage in einem UV-Reaktor bestrahlte Waschflotte mit unbestrahlter Waschflotte aus dem Tank vermischt werden und in eine oder mehrere Kammern der Taktwaschanlage zugeführt werden kann, wobei das Mischungsverhältnis über ein oder mehrere Ventile frei einstellbar ist.

In dieser Ausführungsform kann eine Leitung vom Tank zu den Kammern der Taktwaschanlage führen. Eine weitere Leitung kann vom Tank zu einem UV-Reaktor führen. Vom UV-Reaktor kann eine Leitung wegführen, die sich mit der Leitung, die vom Tank zu den Kammern führt, verbinden kann. In jede der Leitungen kann ein Ventil eingebracht sein. Dabei können die Ventile in den zu den Kammern hinführenden Leitungen jeweils vor der Verbindung der beiden Leitungen angebracht sein. Eine derart beschaffene erfindungsgemäße Taktwaschanlage ermöglicht die genaue Dosierung der bestrahlten Waschflotte.

In einer alternativen Ausführungsform kann das Transportsystem auch so beschaffen sein, dass eine Leitung von einer oder mehreren Kammern zu einem UV-Reaktor führt und eine weitere Leitung von diesem UV-Reaktor wieder zurück zu einer oder mehreren Kammern. Eine derart ausgestaltete erfindungsgemäße Taktwaschanlage ist geeignet, eine Verfahrensführung zu wählen, bei der eine Waschflotte zunächst einer Kammer zugeführt, dieser Kammer entnommen, außerhalb der Kammer bestrahlt und die bestrahlte Flotte erneut der Kammer zugeführt wird. Die Leitungen können Ventile und Pumpen enthalten um den Transport der Flotten zu steuern bzw. zu bewirken.

In einer weiteren Ausführungsform wird die erfindungsgemäße Aufgabe gelöst durch die Verwendung von UV-Strahlung in einem Reinigungsverfahren für Textilien in einer Taktwaschanlage. Bevorzugt weist die UV-Strahlung Anteile im UVC-Bereich auf. Besonders bevorzugt wird ausschließlich UVC-Strahlung verwendet. Die Strahlung kann verwendet werden, um die Waschkraft einer Waschflotte zu verbessern. Bevorzugt wird die UV-Strahlung, insbesondere UVC-Strahlung, in Kombination mit einer Waschflotte, die Wasserstoffperoxid und/oder Persäuren enthält, verwendet.

### Vergleichsbeispiel 1

Ein Waschgangkontrollstreifen nach den Vorgaben gemäß RAL-GZ 992/2 Haushalts- und Objektwäsche wurde in einer Rezeptur aus dem Stand der Technik auf einer Taktwaschanlage gemäß der Fig. unter Einsatz von Wasserstoffperoxid bei 70 °C geprüft. Der Waschgangskontrollstreifen wurde insgesamt 50-fach aufbereitet.

### Waschrezeptur:

| | |
|---|---|
| Vorwäsche: | 2 g/kg Wäsche nichtionogenes Tensid |
| | 2 g/kg Wäsche Akali auf Basis NaOH |
| Hauptwäsche: | 2 g/kg Wäsche Komplexbildner (Phosphonat) |
| | 2 g/kg Wäsche Akali auf Basis NaOH |
| | 4 g/kg Wäsche Wasserstoffperoxid (35%-ig) |
| | 1 g/kg Wäsche opt. Aufheller (Stilbenderivat) |
| pH-Neutralisierung: | 3 g/kg Wäsche Citronensäure |

### Ergebnisse der Standardrezeptur

| Prüfkriterien | | Ergebnisse | Anforderungen nach RAL-GZ 992/2 |
|---|---|---|---|
| Festigkeitsminderung (Reißkraftverlust) | | 19,4% | maximal 30% |
| Schädigungsfaktor (chem. Schädigung) | | 0,3 | maximal 1,0 |
| Glühasche (anorg. Inkrustationen) | | 0,1% | maximal 1,0% |
| Weißqualität | Weißgrad | 205 | minimal 170 |
| | Farbtonabweichung | -0,48 | R 1,50 - G 2,49 |
| | Grundweißwert (Y-Wert) | 88 | minimal 87 |

### Beispiel 1

Ein Waschgangkontrollstreifen nach den Vorgaben gemäß RAL-GZ 992/2 Haushalts- und Objektwäsche wurde in dem erfindungsgemäßen Verfahren auf einer Taktwaschanlage gemäß der Fig. unter Einsatz von Wasserstoffperoxid bei 50 °C und UVC-Bestrahlung der gesamten Waschflotte geprüft. Der Waschgangskontrollstreifen wurde insgesamt 50-fach aufbereitet.

### Waschrezeptur:

| | |
|---|---|
| Vorwäsche: | 2 g/kg Wäsche nichtionogenes Tensid |
| | 1 g/kg Wäsche Akali auf Basis NaOH |
| Hauptwäsche: | 2 g/kg Wäsche Komplexbildner (Phosphonat) |
| | 1 g/kg Wäsche Akali auf Basis NaOH |
| | 2 g/kg Wäsche Wasserstoffperoxid (35%-ig) |
| | 1 g/kg Wäsche opt. Aufheller (Stilbenderivat) |
| pH-Neutralisierung: | 2 g/kg Wäsche Citronensäure |

### Ergebnisse der UV-C Rezeptur

| Prüfkriterien | | Ergebnisse | Anforderungen nach RAL-GZ 992/2 |
|---|---|---|---|
| Festigkeitsminderung (Reißkraftverlust) | | 5,4% | maximal 30% |
| Schädigungsfaktor (chem. Schädigung) | | 0,1 | maximal 1,0 |
| Glühasche (anorg. Inkrustationen) | | 0,1% | maximal 1,0% |
| Weißqualität | Weißgrad | 239 | minimal 170 |
| | Farbtonabweichung | N-0,11 | R 1,50 - G 2,49 |
| | Grundweißwert (Y-Wert) | 92 | minimal 87 |

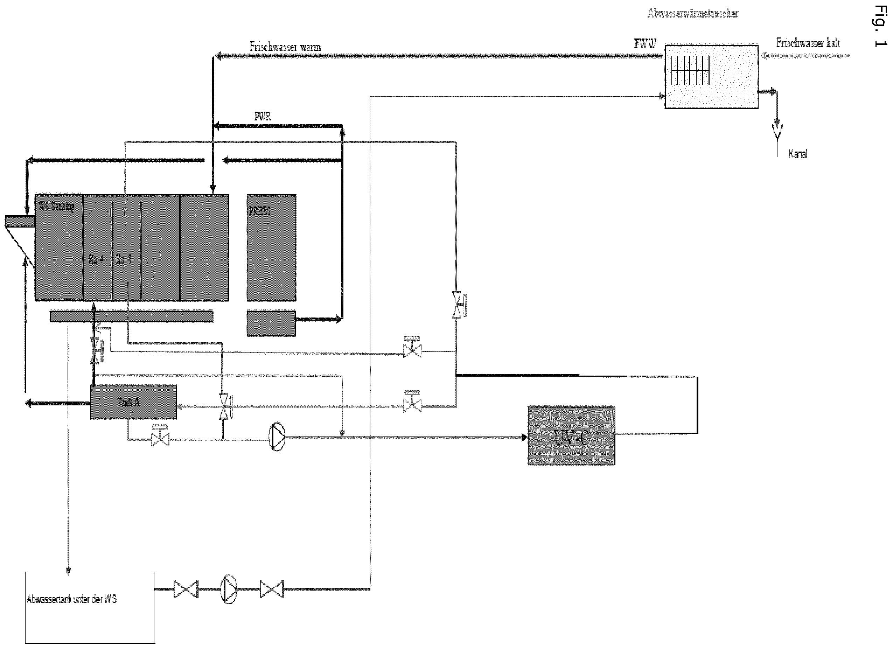

## Patentansprüche

1. Verfahren zum Waschen von Textilien in einer Taktwaschanlage umfassend zwei oder mehr Kammern, wobei die Textilien in einem festgelegten Takt von Kammer zu Kammer transportiert werden, im Gegenstrom Wasser durch die Taktwaschanlage transportiert wird und in eine oder mehrere Kammern eine halogenfreie Waschflotte zugeführt wird, **wobei** die Waschflotte teilweise oder vollständig mit UV-Strahlung bestrahlt wird, **dadurch gekennzeichnet, dass die Waschflotte Wasserstoffperoxid und/oder Persäure in einem Lösungsmittel, insbesondere Wasser, umfasst.**

2. Verfahren nach Anspruch **1**, **dadurch gekennzeichnet, dass** die Persäuren, Peressigsäure, Peroktansäure, Pernonansäure und/oder ε-Phthalamid-Peroxo-Capronsäure einschließlich deren Gemische untereinander sowie mit Wasserstoffperoxid umfassen.

3. Verfahren gemäß einem der Ansprüche **1 oder 2**, **dadurch gekennzeichnet, dass** die Waschflotte ein Lösungsmittel, insbesondere Wasser, sowie ein oder mehrere Stoffe, die ausgewählt sind aus der Gruppe, die aus Tensiden, Alkali, Komplexbildnern, optischen Aufhellern und Bleichmitteln besteht, umfasst.

4. Verfahren gemäß einem oder mehreren der Ansprüche 1 bis **3**, **dadurch gekennzeichnet, dass** die Waschflotte vor der Zuführung in eine oder mehrere Kammern der Taktwaschanlage mit UV-Strahlung bestrahlt wird.

5. Verfahren gemäß einem oder mehreren der Ansprüche 1 bis **4**, **dadurch gekennzeichnet, dass** die eingesetzte UV-Strahlung Anteile im UVC-Bereich aufweist, insbesondere aus UVC-Strahlung besteht.

6. Verfahren gemäß einem oder mehreren der Ansprüche 1 bis **5**, **dadurch gekennzeichnet, dass** das Waschen der Textilien bei einer Temperatur von 40 bis 60 °C, insbesondere bei einer Temperatur von 50 °C, durchgeführt wird.

7. Verfahren gemäß einem oder mehreren der Ansprüche **1** bis **6**, **dadurch gekennzeichnet, dass** die Waschflotte Wasserstoffperoxid in einer Konzentration von 1 bis 5 g/kg Wäsche enthält.

8. Taktwaschanlage zur Durchführung des Verfahrens gemäß einem der Ansprüche 1 bis **7** umfassend zwei oder mehr Kammern, eine Vorrichtung zum getakteten Transport von Textilposten von Kammer zu Kammer, eine Vorrichtung zur Zuführung von Wasser, wobei die Zuführvorrichtung so ausgestaltet ist, dass das Wasser im Gegenstrom zu den Textilposten durch die Kammern transportiert werden kann sowie eine Vorrichtung zur Zuführung einer Waschflotte in eine oder mehrere Kammern umfassend einen oder mehrere Tanks sowie ein Transportsystem, **wobei** die Taktwaschanlage einen oder mehrere UV-Reaktoren umfasst, wobei die UV-Reaktoren entweder a) so in das Transportsystem für die Waschflotte eingebettet sind, dass die Waschflotte ganz oder teilweise aus einem Tank oder einer der Kammern der Taktwaschanlage durch einen oder mehrere UV-Reaktor(en) in die Kammern der Taktwaschanlage geführt werden kann oder b) so angeordnet sind, dass der Innenraum einer oder mehrerer der Kammern der Taktwaschanlage teilweise oder vollständig mit UV-Strahlung bestrahlt werden kann, **dadurch gekennzeichnet, dass der/die die UV-Reaktor(en) UV-Strahlung mit Anteilen im UVC-Bereich erzeugen kann/können.**

9. Taktwaschanlage gemäß Anspr**u**ch **8**, **dadurch gekennzeichnet, dass** das Transportsystem für die Waschflotte so beschaffen ist, dass die vor Zuführung zu einer der Kammern der Taktwaschanlage in einem UV-Reaktor bestrahlte Waschflotte wieder zurück in den Tank geführt werden kann.

10. Taktwaschanlage gemäß einem oder mehreren der Ansprüche **8 oder 9, dadurch gekennzeichnet, dass** das Transportsystem für die Waschflotte so beschaffen ist, dass die vor Zuführung zu einer der Kammern der Taktwaschanlage in einem UV-Reaktor bestrahlte Waschflotte mit einer unbestrahlten Waschflotte aus dem Tank vermischt werden und in eine oder mehrere Kammern der Taktwaschanlage zugeführt werden kann, wobei das Mischungsverhältnis über ein oder mehrere Ventile frei einstellbar ist.

11. Verwendung von UV-Strahlung in einem Reinigungsverfahren für Textilien gemäß einem der Ansprüche 1 bis in einer Taktwaschanlage gemäß einem der Ansprüche 8 bis 10.

12. Verwendung gemäß Anspruch **11**, **dadurch gekennzeichnet, dass** die UV-Strahlung aus UVC-Strahlung besteht.

## Claims

1. A process for laundering textiles in a tunnel washer comprising two or more compartments, wherein the textiles are transported from compartment to compartment in a predetermined pace, water is transported through the tunnel washer in counter-current, and a halogen-free washing liquor is supplied to one or more compartments, wherein said washing liquor is partially or completely irradiated with UV radiation, **characterized in that** said washing liquor comprises hydrogen peroxide and/or peracid in a solvent, especially water.

2. The process according to claim 1, **characterized in that** said peracids include peracetic acid, peroctanoic acid, pernonanoic acid and/or ε-phthalimido peroxycaproic acid including mixtures thereof with one another and with hydrogen peroxide.

3. The process according to any of claims 1 or 2, **characterized in that** said washing liquor comprises a solvent, especially water, and one or more materials selected from the group consisting of surfactants, alkali, complexing agents, optical brighteners and bleaching agents.

4. The process according to one or more of claims 1 to 3, **characterized in that** said washing liquor is irradiated with UV radiation before being supplied to one or more compartments of the tunnel washer.

5. The process according to one or more of claims 1 to 4, **characterized in that** the UV radiation employed comprises radiation in the UVC range, especially consists of UVC radiation.

6. The process according to one or more of claims 1 to 5, **characterized in that** the laundering of the textiles is performed at a temperature of from 40 to 60 °C, especially at a temperature of 50 °C.

7. The process according to one or more of claims 1 to 6, **characterized in that** said washing liquor contains hydrogen peroxide at a concentration of from 1 to 5 g/kg of laundry.

8. A tunnel washer for performing the process according to any of claims 1 to 7, comprising two or more compartments, a device for the timed transport of textile batches from compartment to compartment, a device for supplying water, wherein said supplying device has such a design that the water can be transported in counter-current to the textile batches through the compartments, and a device for supplying a washing liquor to one or more compartments, comprising one or more tanks and a transport system, wherein said tunnel washer comprises one or more UV reactors, wherein said UV reactors either a) are embedded into the transport system for the washing liquor in such a way that the washing liquor can be conducted wholly or in part from the tank or one of the compartments of the tunnel washer through one or more UV reactor(s) into the compartments of the tunnel washer, or b) are arranged in such a way that the interior space of one or more of the compartments of the tunnel washer can be irradiated partially or completely with UV radiation, **characterized in that** said UV reactor(s) can produce UV radiation comprising radiation in the UVC range.

9. The tunnel washer according to claim 8, **characterized in that** said transport system for the washing liquor is of such a nature that the washing liquor having been irradiated in a UV reactor before being supplied to one of the compartments of said tunnel washer can be recirculated into the tank.

10. The tunnel washer according to one or more of claims 8 or 9, **characterized in that** said transport system for the washing liquor is of such a nature that the washing liquor having been irradiated in a UV reactor before being supplied to one of the compartments of said tunnel washer can be mixed with a non-irradiated washing liquor from the tank and supplied to one or more compartments of said tunnel washer, wherein the mixing ratio can be adjusted freely by one or more valves.

11. Use of UV radiation in a laundering process for textiles according to any of claims 1 to 7 in a tunnel washer according to any of claims 8 to 10.

12. The use according to claim 11, **characterized in that** the UV radiation consists of UVC radiation.

## Revendications

1. Procédé pour laver des textiles dans un tunnel de lavage comprenant deux ou plusieurs compartiments, dans lequel les textiles sont transportés d'un compartiment à l'autre à intervalles donnés, de l'eau est transportée à travers ledit tunnel de lavage à contre-courant, et un bain de lavage exempt d'halogène est alimenté dans un ou plusieurs compartiments, dans lequel ledit bain de lavage est irradié avec un rayonnement ultraviolet partiellement ou complètement, **caractérisé en ce que** ledit bain de lavage comprend du peroxyde d'hydrogène et/ou un peracide dans un solvant, notamment de l'eau.

2. Procédé selon la revendication 1, **caractérisé en ce que** lesdits peracides comprennent l'acide peracétique, l'acide peroctanoïque, l'acide pernonanoïque et/ou l'acide ε-phtalamidoperoxycaproïque incluant leurs mélanges entre eux et avec du peroxyde d'hydrogène.

3. Procédé selon l'une des revendications 1 ou 2, **caractérisé en ce que** ledit bain de lavage comprend un solvant, notamment de l'eau, et une ou plusieurs substances choisies dans le groupe consistant en tensioactifs, alcalis, agents complexants, azurants optiques et agents de blanchiment.

4. Procédé selon l'une ou plusieurs des revendications 1 à 3, **caractérisé en ce que** ledit bain de lavage est irradié avec un rayonnement ultraviolet avant d'être alimenté dans un ou plusieurs compartiments dudit tunnel de lavage.

5. Procédé selon l'une ou plusieurs des revendications 1 à 4, **caractérisé en ce que** le rayonnement ultraviolet utilisé présente des parties dans le domaine UVC, notamment est un rayonnement UVC.

6. Procédé selon l'une ou plusieurs des revendications 1 à 5, **caractérisé en ce que** le lavage des textiles est effectué à une température de 40 à 60 °C, notamment à une température de 50 °C.

7. Procédé selon l'une ou plusieurs des revendications 1 à 6, **caractérisé en ce que** ledit bain de lavage contient du peroxyde d'hydrogène dans une concentration de 1 à 5 g/kg de linge.

8. Tunnel de lavage pour la mise en œuvre du procédé selon l'une quelconque des revendications 1 à 7, comprenant deux ou plusieurs compartiments, un dispositif pour le transport à intervalles donnés de lots de textiles d'un compartiment à l'autre, un dispositif pour l'alimentation d'eau, où ledit dispositif d'alimentation est configuré pour transporter l'eau à contre-courant aux lots de textiles à travers les compartiments, et un dispositif pour alimenter un bain de lavage dans un ou plusieurs compartiments, comprenant un ou plusieurs réservoirs et un système de transport, dans lequel ledit tunnel de lavage comprend un ou plusieurs réacteurs à UV, dans lequel lesdits réacteurs à UV sont soit a) incorporés dans ledit système de transport pour le bain de lavage de manière que ledit bain de lavage puisse être guidé complètement ou partiellement d'un réservoir ou d'un des compartiments du tunnel de lavage à travers un ou plusieurs desdits réacteurs à UV dans les compartiments du tunnel de lavage, soit b) arrangés de manière que l'espace intérieur d'un ou de plusieurs des compartiments du tunnel de lavage puissent être irradiés avec un rayonnement ultraviolet partiellement ou complètement, **caractérisé en ce que** ledit ou lesdits réacteurs à UV peuvent produire un rayonnement ultraviolet ayant des parties dans le domaine UVC.

9. Tunnel de lavage selon la revendication 8, **caractérisé en ce que** ledit système de transport pour le bain de lavage est configuré pour recycler dans le réservoir ledit bain de lavage irradié dans un réacteur à UV avant être alimenté dans un des compartiments du tunnel de lavage.

10. Tunnel de lavage selon l'une ou plusieurs des revendications 8 ou 9, **caractérisé en ce que** ledit système de transport pour le bain de lavage est configuré pour mélanger ledit bain de lavage irradié dans un réacteur à UV avant être alimenté dans un des compartiments du tunnel de lavage avec un bain de lavage non irradié provenant dudit réservoir, et pour le recycler dans un ou plusieurs des compartiments du tunnel de lavage, dans lequel le rapport de mélange peut être réglé librement au moyen d'une ou plusieurs vannes.

11. Utilisation de rayonnement ultraviolet dans un procédé pour nettoyer des textiles selon l'une des revendications 1 à 7 dans un tunnel de lavage selon l'une des revendications 8 à 10.

12. Utilisation selon la revendication 11, **caractérisée en ce que** ledit rayonnement ultraviolet est un rayonnement UVC.
